# EUROPEAN PATENT APPLICATION

(11) **EP 4 691 327 A1**
(43) Date of publication of application: **11.02.2026**
(21) Application number: 24781058.3
(22) Date of filing: 07.03.2024
(51) Int. Cl.: A47J 37/06, B01D 46/00

(54) **FILTER HOUSING AND AIR-CIRCULATING ELECTRIC ROASTER INCLUDING SAME**

(30) Priority: 31.03.2023 KR 20230043145; 05.09.2023 JP 2023143499
(71) Applicant: DNW, LTD, Anyang-si, Gyeonggi-do 14084 (KR)
(72) Inventor: KHANG, Hyunho, Jincheon-gun, Chungcheongbuk-do 27839 (KR)
(74) Representative: Tergau & Walkenhorst Intellectual Property GmbH
(86) International application number: PCT/KR2024/002939
(87) International publication number: WO 2024/205065

(57) **Abstract**

A filter member housing includes a first member having a first opening at a first side along a first direction and a first space for accommodating a first side of a filter member, and a second member having a second opening at the first side for receiving the first side of the first member and a second space for accommodating a second side of the filter member and the first side of the first member. A portion of the first side of the first member is inserted into the first side of the second member, so that the filter member is received within a space defined by the first and second spaces. When a protrusion on the first member engages a groove on the second member, the protrusion is positioned at a first end of the groove by the elastic force of the filter member. Applying pressure moves the protrusion along the groove to a second end, and releasing pressure allows the protrusion to return to the first end by the elastic force, thereby securing the filter member in place.

## Description

### [Technical Field]

The present invention relates to a filter member housing and an air-circulating electric roaster including the same.

### [Background Art]

An air-circulating electric roaster, in which an elongated fan is arranged on one side of the lower part of the main body and a cooking part is arranged on the upper part, is configured such that when cooking, the fan operates to form a constant airflow inside the electric roaster so that smoke is not discharged to the outside, and oil is collected in an oil tray inside through an oil hole of the cooking part.

In such an air-circulating electric roaster, an air intake portion and an air discharge portion are respectively formed at side surfaces of an air circulation plate, and as a fan operates, air is drawn from a cooking part, passes through a lower portion of the air circulation plate, and is discharged through the air discharge portion.

To solve the problems of fine odors still being emitted to the outside from an electric roaster with such a structure, and oil contained in the air sucked during cooking adhering to the intake passage and fan inside the electric roaster before passing through the oil tray, causing internal contamination, which leads to frequent cleaning and difficulty in removing oil stains, an air-circulating electric roaster using a cross-flow fan has been disclosed that can minimize contamination of the air intake passage and the cross-flow fan without hindering smooth air circulation (see, for example, Japanese Patent No. 7081870).

In the case of the air-circulating electric roaster disclosed in Japanese Patent No. 7081870, when a plate-shaped odor attenuation member is arranged on the side of the main body, a porous material member is inserted into a narrow side space and fixed by a predetermined fixing part.

When used alone, a porous material member is easy to insert into a narrow space, but it requires a fixing part to fix it, and when oil molecules are adsorbed and contaminated, there is the inconvenience of having to touch the contaminated porous material member itself to remove them.

If a housing is used to accommodate the porous material member to solve this problem, it is difficult to insert the housing into a narrow space where the front is not open, and if the size of the housing is made sufficiently small to facilitate insertion, a fixing part is still required to fix it.

Hereinafter, in this specification, a porous material member including such an odor attenuation member is referred to as a "filter member".

### [Disclosure]

### [Technical Problem]

The present invention has been made in view of the above aspects, and it is an object of the present invention to provide a filter member housing that can be easily attached and detached in a narrow space and can be self-fixed without a fixing part.

It is another object of the present invention to provide an air-circulating electric roaster equipped with a filter member housing that is self-fixed without a fixing part to effectively attenuate oil and odors contained in the circulating air in the air-circulating electric roaster.

The problems to be solved by the present invention are not limited to those mentioned above, and other problems not mentioned will be clearly understood by those skilled in the art from the following description.

### [Means for Solving Problem]

According to at least one embodiment of the present invention, a filter member housing includes a first member having a first opening formed at a first side along a first direction and a first space for accommodating at least a first side of a filter member through the first opening and a second member having a second opening formed at the first side along the first direction so that the first side of the first member is inserted therein, and a second space for accommodating at least a second side of the filter member and the first side of the first member through the second opening. The first member and the second member are configured to be coupled in a detachable manner, and form a receiving space surrounded by the first space and the second space by inserting a portion of the first side of the first member into the first side of the second member. The filter member is accommodated in the receiving space by accommodating the first side of the filter member in the first space of the first member or the second side of the filter member in the second space of the second member, and then accommodating the second side of the filter member in the second space of the second member while inserting a portion of the first side of the first member into the first side of the second member or accommodating the first side of the filter member in the first space of the first member while inserting a portion of the first side of the first member into the first side of the second member. The first member includes a protrusion formed on a portion of the first side that is inserted into the first side of the second member. The second member includes a groove formed at a position corresponding to the protrusion and configured to allow the protrusion to slide in a second direction orthogonal to the first direction when the portion of the first side of the first member is inserted into the first side of the second member. When the filter member is accommodated in the receiving space and the portion of the first side of the first member is inserted into the first side of the second member so that the protrusion is engaged with the groove, the protrusion is positioned at a first end of the groove by an elastic force of the filter member, and when pressure is applied in the second direction to the first member relative to the second member or to the second member relative to the first member, the protrusion moves along the groove toward a second end of the groove, and when the pressure is released, the protrusion returns to the first end of the groove by elastic recovery of the filter member, thereby being fixed in the groove.

Furthermore, according to at least one embodiment of the present invention, a filter member housing includes a first member having a first opening formed at a first side along a first direction and a first space for accommodating at least a first side of a filter member through the first opening and a second member having a second opening formed at the first side along the first direction so that the first side of the first member is inserted therein, and a second space for accommodating at least a second side of the filter member and the first side of the first member through the second opening. The first member and the second member are coupled such that a portion of the first side of the first member is inserted into the first side of the second member, thereby defining a receiving space surrounded by the first space and the second space. The first member and the second member, when coupled, comprise a lateral opening having one side open along a second direction perpendicular to the first direction. The filter member is inserted through the lateral opening and accommodated in the receiving space when the first member and the second member are coupled. The first member includes a protrusion formed on a portion of the first side that is inserted into the first side of the second member. The second member includes a groove formed at a position corresponding to the protrusion, the groove being configured to allow the protrusion to slide in the second direction when the portion of the first side of the first member is inserted into the first side of the second member. When the filter member is accommodated in the receiving space with the first and second members coupled, the protrusion is positioned at a first end of the groove by the elastic force of the filter member, and when pressure is applied to the first member relative to the second member or to the second member relative to the first member along the second direction, the protrusion moves along the groove toward a second end of the groove, and when the pressure is released, the protrusion returns to the first end of the groove by the elastic recovery of the filter member, thereby being fixed in the groove.

Moreover, according to at least one embodiment of the present invention, a filter member housing includes a first member having a first opening formed at a first side along a first direction and a first space for accommodating at least a first side of a filter member through the first opening, a second member having a second opening formed at the first side along the first direction so that the first side of the first member is inserted therein, and a second space for accommodating at least a second side of the filter member and the first side of the first member through the second opening, a sliding groove formed in the second member along a second direction perpendicular to the first direction at the first side, the sliding groove allowing sliding and mutual rotation of the first member and the second member when coupled, and a rotating shaft protrusion formed on the first member and inserted from an inner side of the second member into the sliding groove so as to slide along the sliding groove. The first member and the second member are mutually rotated about the rotating shaft protrusion to insert a portion of the first side of the first member into the first side of the second member, thereby defining a receiving space surrounded by the first space and the second space. The filter member is accommodated in the receiving space by inserting its first side into the first space of the first member or its second side into the second space of the second member, and subsequently inserting a portion of the first side of the first member into the first side of the second member. The first member includes a protrusion formed on a portion of the first side that is inserted into the first side of the second member. The second member includes a groove formed at a position corresponding to the protrusion, the groove being configured to allow the protrusion to slide along the second direction when the portion of the first side of the first member is inserted into the first side of the second member. When the filter member is accommodated in the receiving space and the portion of the first side of the first member is inserted into the first side of the second member so that the protrusion is engaged with the groove, the protrusion is positioned at a first end of the groove by the elastic force of the filter member, when pressure is applied to the first member relative to the second member or to the second member relative to the first member along the second direction, the protrusion moves along the groove toward a second end of the groove, and when the pressure is released, the protrusion returns to the first end of the groove by the elastic recovery of the filter member, thereby being fixed in the groove.

Furthermore, according to at least one embodiment of the present invention, the first member or the second member further includes at least one handle for applying pressure to the second member or the first member along the second direction.

Moreover, according to at least one embodiment of the present invention, at least one of the first member and the second member includes at least one window opened so that a first surface of the filter member is exposed when the filter member is accommodated in the receiving space.

Furthermore, according to at least one embodiment of the present invention, the filter member is accommodated within an installation space having a length in the second direction between a first position, at which the protrusion is positioned at the first end of the groove by the elastic force of the filter member when the filter member is accommodated in the receiving space with the first and second members coupled, and a second position, at which the protrusion moves to the second end of the groove when pressure is applied, and during installation, the first member or the second member is first inserted into the installation space, followed by pressing the second member against the first member or the first member against the second member, and upon releasing the pressed member, the protrusion returns to the first end of the groove by elastic recovery of the filter member, thereby self-fixing the filter member in the installation space.

Moreover, according to at least one embodiment of the present invention, the filter member housing further includes an anti-slip member formed on a lower side of the second member.

Furthermore, according to at least one embodiment of the present invention, an air-circulating electric roaster includes a body, a heater for generating heat, a fan disposed inside the body along a first direction, a cooking plate disposed above the heater and heated by the heat from the heater, an air intake part having at least one hole for drawing air above the cooking plate into the body, an air discharge part having at least one hole for discharging air passing through the body toward the top of the cooking plate, and the filter member housing according to at least one embodiment of the present invention.

Although each embodiment is described independently in this specification, each embodiment can be combined with each other, and the combined embodiments are also within the scope of the present invention.

The foregoing summary is for illustrative purposes only and is not intended to be limiting in any way. In addition to the descriptive aspects, embodiments, and features described above, further aspects, embodiments, and features will become apparent with reference to the drawings and the detailed description below.

### [Advantageous Effects]

According to at least one embodiment of the present invention, there is an effect of providing a filter member housing that is easy to attach and detach in a narrow space and can be self-fixed without a fixing part.

Furthermore, with another technical goal to be achieved by the present invention, there is an effect of providing an air-circulating electric roaster equipped with a filter member housing that is self-fixed without a fixing part to effectively attenuate oil and odors contained in the circulating air of the air-circulating electric roaster.

The effects of the present invention are not limited to those mentioned above, and other effects not mentioned will be clearly understood by those skilled in the art from the following description.

### [Brief Description of Drawings]

FIG. 1 is a perspective view of a filter member housing according to at least one embodiment of the present invention.
FIG. 2 is a front view and a rear view of a filter member housing according to at least one embodiment of the present invention.
FIG. 3 is a cross-sectional perspective view of an air-circulating electric roaster equipped with a filter member housing according to at least one embodiment of the present invention.
FIG. 4 is a front view, top view, bottom view, and left and right side views of a filter member housing according to at least one embodiment of the present invention.
FIG. 5 is a front view and a right side view for explaining the operation of a filter member housing according to at least one embodiment of the present invention.
FIG. 6 is a front view and a right side view for explaining the relationship between the first member and the second member of a filter member housing and the filter member accommodated therein according to at least one embodiment of the present invention.
FIG. 7 is a perspective view for explaining the relationship between the first member and the second member of a filter member housing and the filter member accommodated therein according to at least one embodiment of the present invention.
FIG. 8 is a perspective view for explaining the process of mounting a filter member housing on an air-circulating electric roaster according to at least one embodiment of the present invention.
FIG. 9 is a side cross-sectional view of an air-circulating electric roaster equipped with a filter member housing according to at least one embodiment of the present invention.
FIG. 10 is a front view, top view, bottom view, and left and right side views of a filter member housing according to at least one embodiment of the present invention.
FIG. 11 is a perspective view of a filter member housing according to at least one embodiment of the present invention.
FIG. 12 is a perspective view showing the open state of a filter member housing according to at least one embodiment of the present invention.
FIG. 13 is a perspective view of a filter member housing according to at least one embodiment of the present invention.
FIG. 14 is a front view, top view, bottom view, and left and right side views of a filter member housing according to at least one embodiment of the present invention.
FIG. 15 is a side cross-sectional view of an air-circulating electric roaster equipped with a filter member housing according to at least one embodiment of the present invention.

### [Detailed Description]

Hereinafter, embodiments of the present invention will be described in detail with reference to the accompanying drawings.

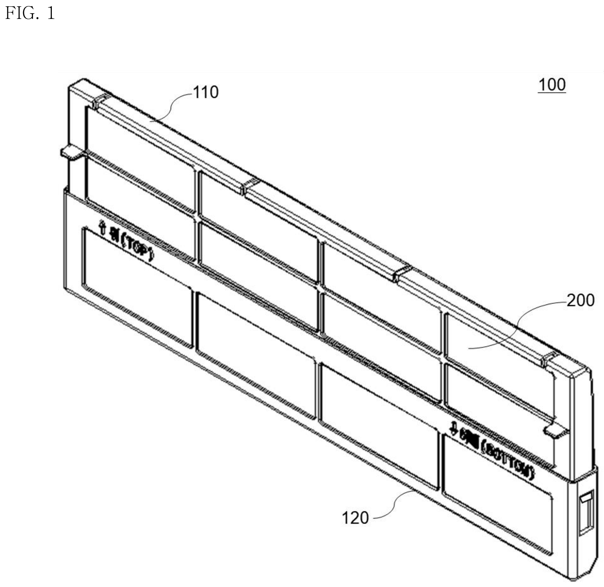

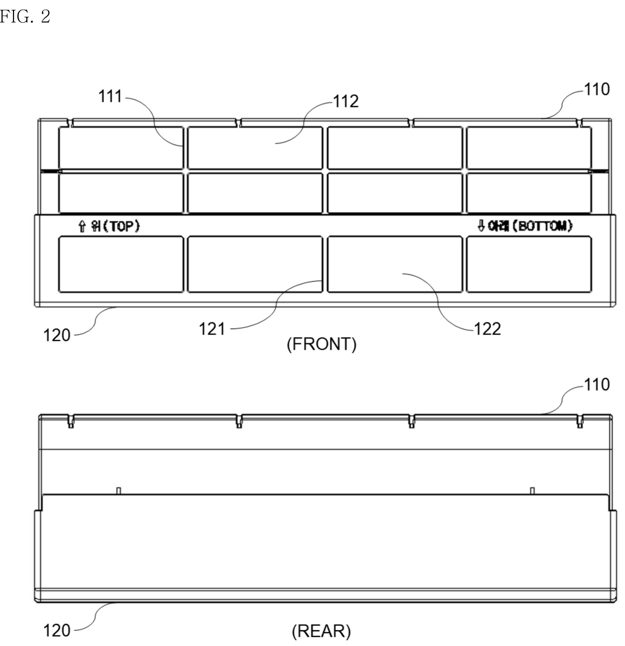

FIG. 1 is a perspective view of a filter member housing 100 according to at least one embodiment of the present invention. FIG. 2 is a front view and a rear view of the filter member housing 100.

As shown in FIG. 1, the filter member housing 100 according to at least one embodiment of the present invention is formed in an overall flat plate shape with a filter member 200 inserted into the internal space between the front part having an open window and the rear part blocked forming a wall.

In at least one embodiment of the present invention, the filter member 200 is a general term for a porous material member including an odor attenuation member, an air purification member, or a wall contamination prevention member.

As shown in FIG. 2, the filter member housing 100 includes a first member 110 forming the upper part and a second member 120 forming the lower part, and the first member 110 and the second member 120 are configured to be freely combinable and separable.

The filter member housing 100 according to at least one embodiment of the present invention can, for example, be mounted on the side of the fan of an air-circulating electric roaster to solve the problem of fine odors still being emitted to the outside and the problem of internal contamination caused by oil contained in the air sucked during cooking adhering to the intake passage and fan inside the electric roaster before passing through the oil tray.

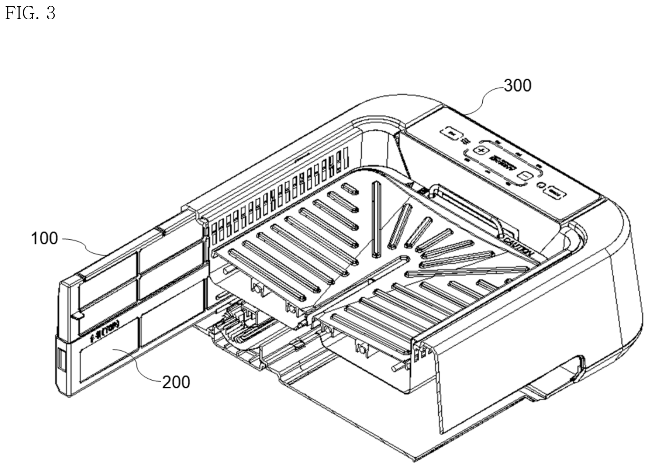

FIG. 3 is a cross-sectional perspective view of an air-circulating electric roaster 300 equipped with the filter member housing 100.

As shown in FIG. 3, the filter member housing 100 according to at least one embodiment of the present invention can be stably mounted in a narrow space on the side of the air-circulating electric roaster 300 without a separate fixing part.

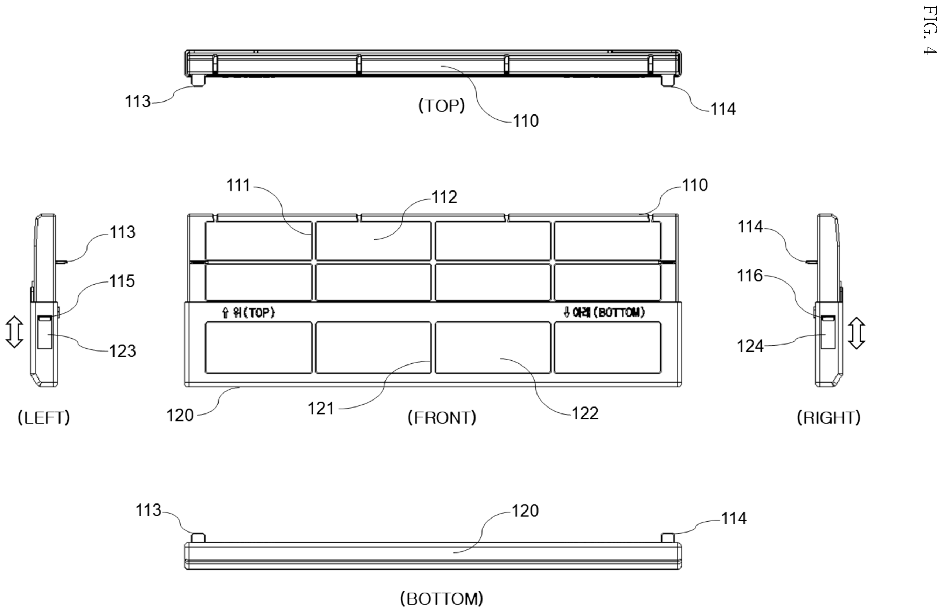

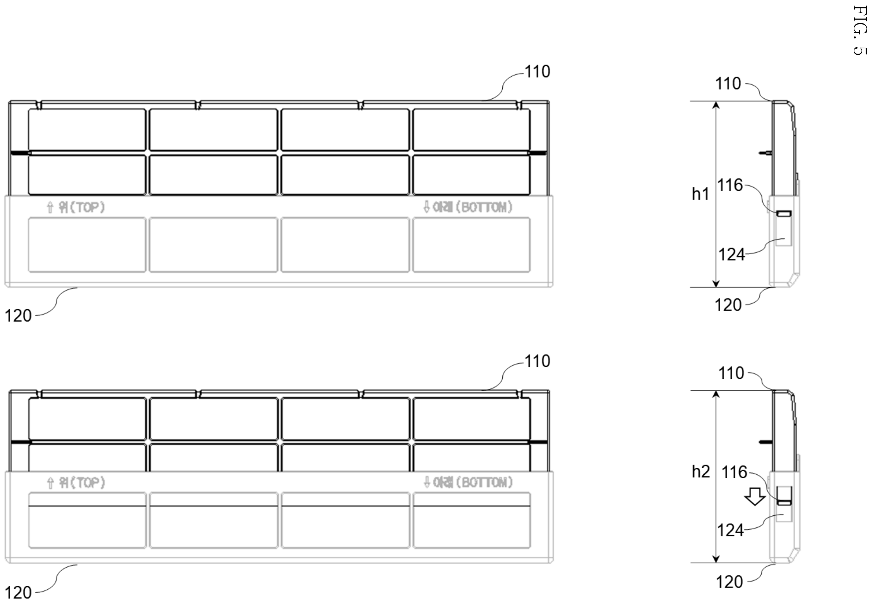

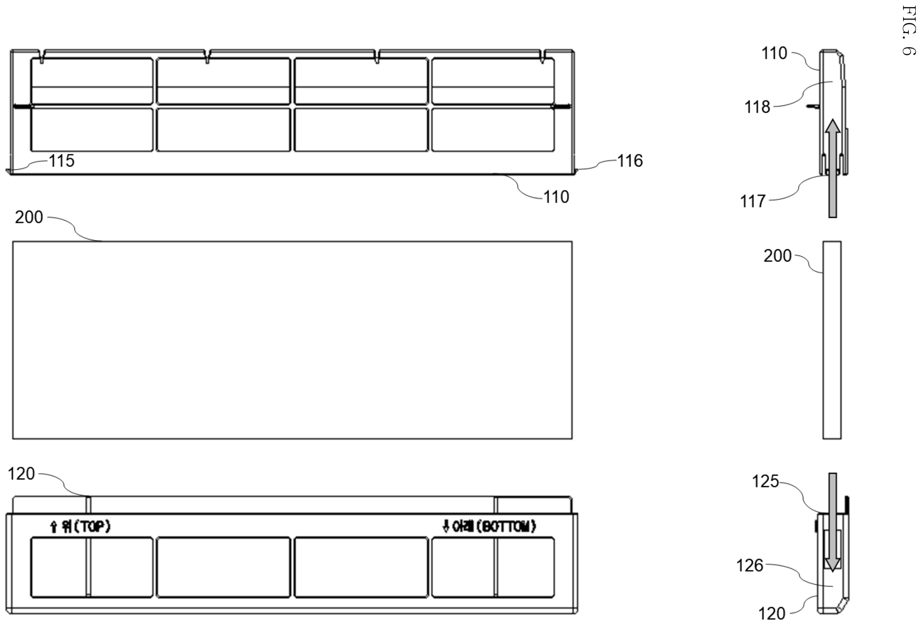

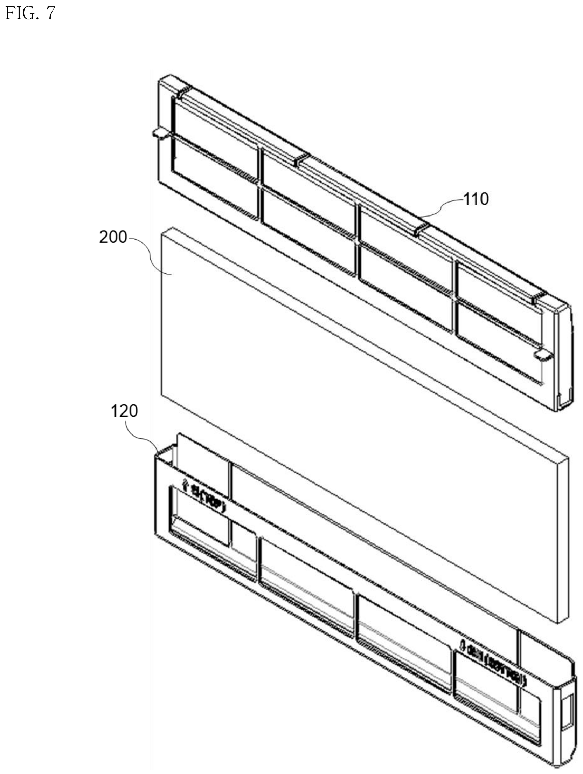

FIG. 4 is a front view, top view, bottom view, and left and right side views of the filter member housing 100. FIG. 5 is a front view and a right side view for explaining the operation of the filter member housing 100. FIG. 6 is a front view and a right side view for explaining the relationship between the first member 110 and the second member 120 of the filter member housing 100 and the filter member 200 accommodated therein according to at least one embodiment of the present invention. FIG. 7 is a perspective view for explaining the relationship between the first member 110 and the second member 120 of the filter member housing 100 and the filter member 200 accommodated therein according to at least one embodiment of the present invention.

As shown in FIGS. 4 to 7, the filter member housing 100 according to at least one embodiment of the present invention includes the first member 110 including a first opening 117 formed on a first side along a first direction and a first space 118 for accommodating at least a first side of the filter member 200 through the first opening 117, and the second member 120 including a second opening 125 formed on a first side along the first direction so that the first side of the first member 110 is inserted, and a second space 126 for accommodating at least a second side of the filter member 200 and the first side of the first member 110 through the second opening 125.

The first direction refers to the horizontal direction, which is the lengthwise direction of the filter member housing 100 in the front view shown in FIG. 2, and the second direction mentioned hereinafter refers to the vertical direction, which is the coupling direction of the filter member housing 100 in the front view shown in FIG. 2.

Therefore, the first side of the first member 110 refers to the lower side of the first member 110 facing the filter member 200 in the front view shown in FIG. 6, and the first side of the second member 120 refers to the upper side of the second member 120 facing the filter member 200 in the front view shown in FIG. 6. Hereinafter, the 'second side' refers to the side opposite to the first side.

In at least one embodiment of the present invention, the first member 110 and the second member 120 are configured to be separable and combinable, and by inserting a part of the first side of the first member 110 into the first side of the second member 120, a receiving space enclosed by the first space 118 and the second space 126 is formed.

The filter member 200 is accommodated in the receiving space by accommodating the first side in the first space 118 of the first member 110 or the second side in the second space 126 of the second member 120, and then inserting a part of the first side of the first member 110 into the first side of the second member 120 while accommodating the second side of the filter member 200 in the second space 126 of the second member 120, or by inserting a part of the first side of the first member 110 into the first side of the second member 120 while accommodating the first side of the filter member 200 in the first space 118 of the first member 110.

When the filter member housing 100 is formed by inserting the first side of the first member 110 into the first side of the second member 120, the filter member 200 is accommodated in the receiving space formed inside. At this time, as shown in FIG. 2, the front surface is exposed to the outside by a first window 112 formed by a first grille 111 of the first member 110 and a second window 122 formed by a second grille 121 of the second member 120, and the rear surface is blocked like a wall.

Therefore, as shown in FIG. 3, when the filter member housing 100 is mounted such that its rear surface faces the wall surface of the main body, oil molecules are primarily adsorbed by the exposed filter member 200 on the front surface, and oil molecules passing through it are blocked inside the rear surface of the filter member housing 100, thereby preventing contamination of the wall surface and fan of the air-circulating electric roaster 300 by oil molecules.

In at least one embodiment of the present invention, the first member 110 includes protrusions (first protrusion 115 and second protrusion 116) formed on a part of the portion inserted into the first side of the second member 120. Although FIGS. 4 to 6 show the first protrusion 115 and the second protrusion 116 formed on both the left and right sides of the first member 110, respectively, this is just one embodiment, and the position of the protrusions can be formed at any position on the lower side of the first member 110, i.e., on the part inserted into the first side of the second member 120.

In at least one embodiment of the present invention, the second member 120 includes grooves (first groove 123 and second groove 124) formed at positions corresponding to the protrusions 115, 116, configured such that when a part of the first side of the first member 110 is inserted into the first side of the second member 120, the protrusions 115, 116 are coupled and can slide in a second direction perpendicular to the first direction. Although FIGS. 4 and 5 show the first groove 123 and the second groove 124 formed on both the left and right sides of the second member 120, respectively, at positions corresponding to the first protrusion 115 and the second protrusion 116, this is based on the premise that the first protrusion 115 and the second protrusion 116 are formed on the first member 110, and they can be formed at corresponding positions depending on the position of the protrusions formed on the first member 110.

In at least one embodiment of the present invention, the protrusions (at least one of the first protrusion 115 and the second protrusion 116) function as a release member to allow the first member 110 to be separated from the second member 120 by pressing this part when the filter member housing 100 is opened (to accommodate the filter member 200 in the receiving space formed inside or to remove the filter member 200 accommodated in the receiving space by separating the first member 110 and the second member 120).

In at least one embodiment of the present invention, when the filter member 200 is accommodated in the receiving space and a part of the first side of the first member 110 is inserted into the first side of the second member 120 and the protrusions 115, 116 are coupled into the grooves 123, 124, with the protrusions 115, 116 located at the first end of the grooves 123, 124 due to the elastic force of the filter member 200, if pressure is applied to the first member 110 against the second member 120 or to the second member 120 against the first member 110 along the second direction, the protrusions 115, 116 move along the grooves 123, 124 towards the second end of the grooves 123, 124, and when the pressure is released, the protrusions 115, 116 return to the position of the first end of the grooves 123, 124 due to the elastic restoring force of the filter member 200.

That is, the first end of the grooves 123, 124 is the position where the vertical distance is maximized when the first member 110 and the second member 120 are coupled, and the second end of the grooves 123, 124 is the position where the vertical distance is minimized when the first member 110 and the second member 120 are coupled.

In at least one embodiment of the present invention, the first member 110 or the second member 120 further includes at least one handle (first handle 113 and second handle 114) for applying pressure to the second member 120 or the first member 110, respectively, along the second direction. Although FIGS. 4 and 7 show the first handle 113 and the second handle 114 formed on the front of both the left and right sides of the first member 110, respectively, this is merely one embodiment, and a handle may alternately be formed at any position that is not inserted into the first side of the second member 120, i.e., at a certain distance upwards from the lower side of the first member 110.

In at least one embodiment of the present invention, at least one of the first member 110 and the second member 120 includes at least one window 112 or 122 formed as an opening such that the first surface of the filter member 200 is exposed to the outside when the filter member 200 is accommodated in the receiving space.

In at least one embodiment of the present invention, the filter member 200 is configured to be installed within an installation space, which is between a first position, where the protrusion 115, 116 is located at the first end of the groove 123, 124 due to the elastic force of the filter member 200 when the filter member 200 is accommodated in the receiving space in the mutually coupled state of the first member 110 and the second member 120, and a second position, where the protrusion 115, 116 moves along the groove 123, 124 towards the second end of the groove 123, 124 when pressure is applied to the first member 110 against the second member 120 or to the second member 120 against the first member 110 along the second direction.

That is, in at least one embodiment of the present invention, when the filter member 200 is accommodated in the receiving space of the filter member housing 100, the filter member 200 has a length in the second direction that exerts an elastic force such that the protrusions 115, 116 touch the first end of the grooves 123, 124 or move further upwards when no force is applied in the second direction, and can be compressed enough for the protrusions 115, 116 to touch the second end of the grooves 123, 124 when force is applied in the second direction.

In at least one embodiment of the present invention, the protrusions 115, 116 are formed on at least one side surface of the first member 110, along the second direction, which is inserted into the first side of the second member 120 (at least one of the first protrusion 115 and the second protrusion 116).
Accordingly, the grooves 123, 124 are formed at positions corresponding to the protrusions 115, 116 on at least one side surface of the second member 120, along the second direction (at least one of the first groove 123 and the second groove 124).

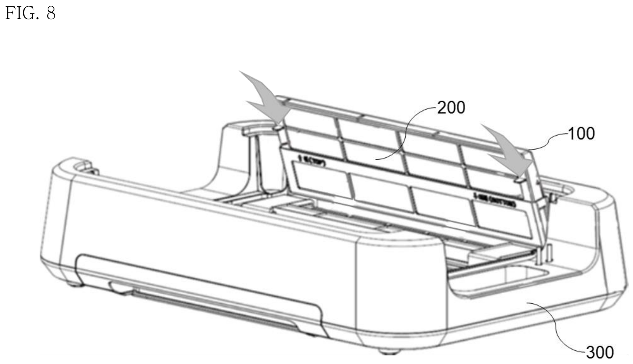

FIG. 8 is a perspective view for explaining the process of mounting the filter member housing 100 on the air-circulating electric roaster 300 according to at least one embodiment of the present invention.

In at least one embodiment of the present invention, when installing, the first member 110 or the second member 120 is first placed into the installation space, and then the second member 120 or the first member 110 is inserted by pressing the second member 120 against the first member 110 or the first member 110 against the second member 120 using the handles 113, 114, and then when the handles 113, 114 are released, the protrusions 115, 116 return to the position of the first end of the grooves 123, 124 due to the elastic restoring force of the filter member 200, thereby being self-fixed within the installation space.

That is, as shown in FIG. 8, the filter member housing 100, in which the filter member 200 is accommodated inside and the first protrusion 115 and the second protrusion 116 are located at the first end of the first groove 123 and the second groove 124 respectively, is oriented towards the inside of the air-circulating electric roaster 300, and the second member 120, which is the lower part of the filter member housing 100, is placed into the installation space. Then, while pressing the first handle 113 and the second handle 114, the first member 110, which is the upper part of the filter member housing 100, is pressed downwards along the side of the air-circulating electric roaster 300, thereby allowing the filter member housing 100 to be inserted in parallel with the side of the air-circulating electric roaster 300.

When the filter member housing 100 is inserted parallel to the side of the air-circulating electric roaster 300, and the first handle 113 and the second handle 114 are released, the lower side of the second member 120 touches the side bottom of the air-circulating electric roaster 300 and the upper side of the first member 110 touches the side ceiling of the air-circulating electric roaster 300, and the filter member housing 100 is self-fixed within the installation space due to the elastic force of the filter member 200.

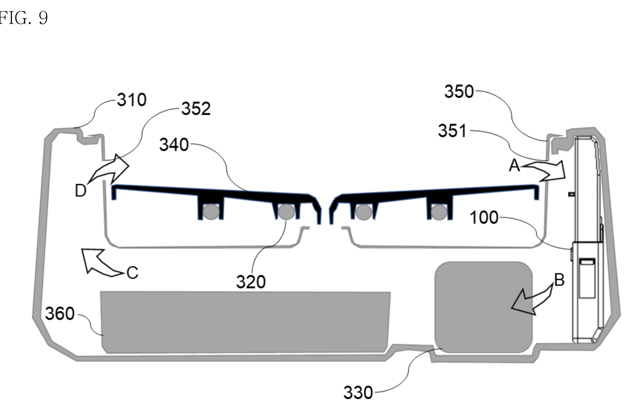

FIG. 9 is a side cross-sectional view of the air-circulating electric roaster 300 equipped with the filter member housing 100 according to at least one embodiment of the present invention.

As shown in FIG. 9, the air-circulating electric roaster 300 according to at least one embodiment of the present invention includes a body 310, a heater 320 for generating heat, a fan 330 disposed inside the body 310 along a first direction of the body 310, a cooking plate 340 disposed above the heater 320 and heated by heat from the heater 320, an air intake part 351 having one or more holes for sucking air from the upper part of the cooking plate 340 towards the body 310, an air discharge part 352 having one or more holes for discharging air that has passed through the inside of the body 310 towards the upper part of the cooking plate 340, and the filter member housing 100 for disposing the filter member 200, for purifying the air sucked through the air intake part 351, on the inner wall side of the body 310, with its widest surface oriented parallel to the air flow path inside the body 310.

In at least one embodiment of the present invention, the filter member 200 is a member that has the function of minimizing contamination of the air intake passage and the cross-flow fan without hindering smooth air circulation in the air-circulating electric roaster 300 using a cross-flow fan, and at the same time, in the circulating air of the air-circulating electric roaster 300 using a cross-flow fan.

In addition, the filter member 200 minimizes contamination of the wall surface and the fan 330 of the air-circulating electric roaster 300 by oil molecules.

The body 310 is placed on a floor such as a table or the ground, incorporates various components therein to form the overall external appearance of the air-circulating electric roaster 300 according to at least one embodiment of the present invention, and has the fan 330 positioned inside a lower portion of the body 310, with an oil tray 360, an air circulation plate 350, the heater 320, and the cooking plate 340 sequentially arranged from bottom to top.

In at least one embodiment of the present invention, the fan 330 mounted inside the body 310 is an elongated fan having a built-in motor, and by operating the fan 330 to circulate air inside, smoke (hot air containing oil and odor) generated during cooking is drawn in, so that only air from which oil and odor have been removed is discharged to the outside of the body 310.

In at least one embodiment of the present invention, the air intake part 351 and the air discharge part 352 are formed on the side of the air circulation plate 350. When the fan 330 is operated during cooking, air (smoke) containing oil and odor is drawn in through the air intake part 351, passes over the oil tray 360 beneath the air circulation plate 350 and is discharged to the upper side of the cooking plate 340 through the air discharge part 352 (see arrows A, B, C, and D in FIG. 9).

In an air-circulating electric roaster using a cross-flow fan, in order to minimize contamination of the air intake passage and the cross-flow fan without hindering smooth air circulation and to effectively attenuate oil and odor contained in the circulating air of the air-circulating electric roaster using a cross-flow fan, as shown in FIG. 9, the air-circulating electric roaster 300 according to at least one embodiment of the present invention includes the filter member housing 100 mounted in a side space where the fan 330 is located.

In the internal space of the filter member housing 100, the filter member 200 is accommodated. The filter member 200 is a plate-shaped porous material member having a predetermined thickness, width, and height, and is self-fixed within the side space of the inner side of the body 310 without a separate fixing member.

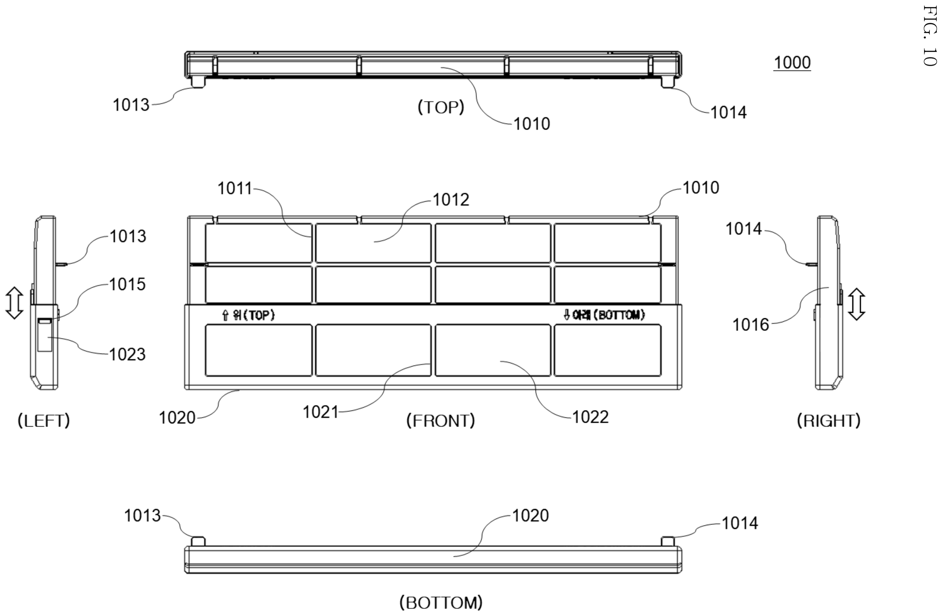

FIG. 10 is a front view, a top view, a bottom view, and left and right side views of a filter member housing 1000 according to at least one embodiment of the present invention.

While the filter member housing 100 illustrated in FIGS. 3 to 6 has closed left and right side surfaces and is configured such that the upper side of the filter member 200 is inserted into the first member 110 and the lower side is inserted into the second member 120 while the first member 110 and the second member 120 are separated and then coupled together, the filter member housing 1000 shown in FIG. 10 has a structure in which the filter member 200 can be freely inserted and removed through an open side slot with the first member and the second member already coupled together.

To this end, the filter member housing 1000 according to at least one embodiment of the present invention includes a first member 1010 having a first opening formed on a first side along a first direction and a first space for accommodating at least a first side of the filter member 200 through the first opening, and a second member 1020 having a second opening formed on the first side along the first direction, which is configured to receive the first side of the first member 1010, and a second space for accommodating at least a second side of the filter member 200 and the first side of the first member 1010 through the second opening.

In at least one embodiment of the present invention, the first member 1010 and the second member 1020 are coupled such that a portion of the first side of the first member 1010 is inserted into the first side of the second member 1020, thereby defining a receiving space jointly defined by the first space and the second space.

In at least one embodiment of the present invention, the first member 1010 and the second member 1020 include a lateral opening 1016 that is open on one side along a second direction perpendicular to the first direction while being coupled to each other. The filter member 200 is inserted through the lateral opening 1016 and accommodated in the receiving space while the first member 1010 and the second member 1020 are coupled to each other.

In at least one embodiment of the present invention, the first member 1010 includes a projection 1015 formed on a portion of the first side that is inserted into the first side of the second member 1020. The second member 1020 includes a groove (first groove 1023) formed at a position corresponding to the projection 1015, the groove being configured to couple with the projection 1015 and allow sliding in the second direction when a portion of the first side of the first member 1010 is inserted into the first side of the second member 1020.

In at least one embodiment of the present invention, when the filter member 200 is accommodated in the receiving space while the first member 1010 and the second member 1020 are coupled to each other, the projection 1015 is positioned at a first end of the groove 1023 by the resilient force of the filter member 200. When pressure is applied to the first member 1010 relative to the second member 1020 or to the second member 1020 relative to the first member 1010 along the second direction, the projection 1015 moves along the groove 1023 toward the second end of the groove 1023, and when the pressure is released, the projection 1015 is restored to the position at the first end of the groove 1023 by the resilient restoring force of the filter member 200.

In at least one embodiment of the present invention, the projection 1015 is formed on a portion of the first side of the first member 1010 that is inserted into the first side of the second member 1020, on the side opposite to the lateral opening 1016.

Since the function and operation of the filter member housing 1000 are similar to those of the filter member housing 100, a detailed description thereof will be omitted.

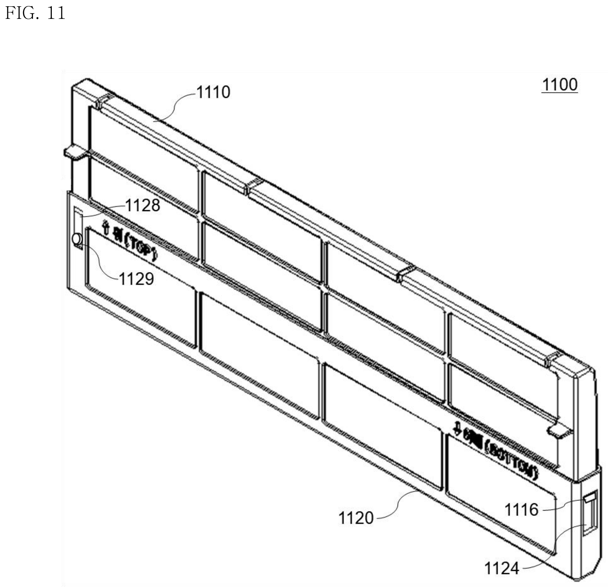

FIG. 11 is a perspective view of a filter member housing 1100 according to at least one embodiment of the present invention.

The filter member housing 100 illustrated in FIG. 1 accommodates the filter member 200 in a receiving space, which is defined by the first space 118 and the second space 126 by coupling the separate first member 110 and second member 120, as shown in FIGS. 6 and 7. When removing the filter member 200 accommodated in the receiving space, the coupled first member 110 and second member 120 are configured to be separable.

On the other hand, in the filter member housing 1100 illustrated in FIG. 11, a sliding groove 1128 is formed in a second member 1120 to allow a first member 1110 and the second member 1120, which are coupled at the first side, to rotate. A rotation shaft projection 1129 is formed on the first member 1110, which is inserted into the sliding groove 1128 from the inside of the second member 1120 and slides along the sliding groove 1128.

On the second side opposite the first side, a projection 1116 is formed on the first member 1110, and a groove 1124 is formed on the second member 1120 to couple with the projection 1116 of the first member 1110.

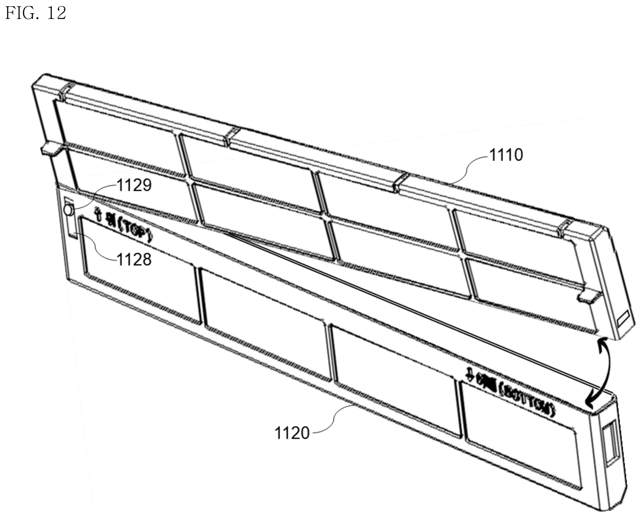

FIG. 12 is a perspective view illustrating the open state of the filter member housing 1100 shown in FIG. 11.

As shown in FIG. 12, the filter member housing 1100 allows the first member 1110 and the second member 1120 to rotate relative to each other about the rotation shaft projection 1129, thereby opening or closing the internal receiving space. When closed, the projection 1116 of the first member 1110 is configured to be coupled into the groove 1124.

When opened, the projection 1116 of the first member 1110 acts as a release member that allows the second sides of the first member 1110 and the second member 1120 to be separated by pressing the projection 1116 to disengage it from the groove 1124.

The upper corner portion of the first side of the second member 1120 may include a recess of a predetermined depth to provide space when the first member 1110 rotates about the rotation shaft projection 1129.

As another configuration for allowing the first member 1110 and the second member 1120 to rotate about the rotation shaft projection 1129, a bent portion may be formed on the upper part of the rotation shaft projection 1129 of the first member 1110. In this configuration, the rotation shaft projection 1129 slides along the sliding groove 1128, while the first member 1110 rotates along the bent portion.

By allowing one side of the first member 1110 and the second member 1120 to rotate, the filter member 200 can be accommodated in or removed from the internal receiving space without completely separating the first member 1110 and the second member 1120, thereby improving ease of use.

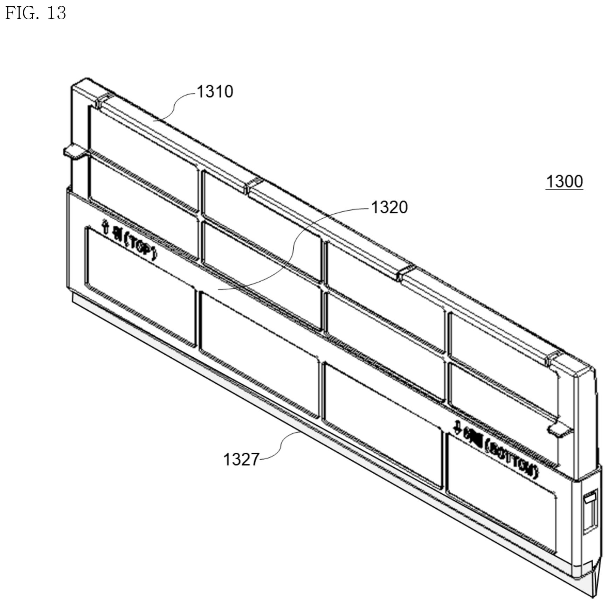

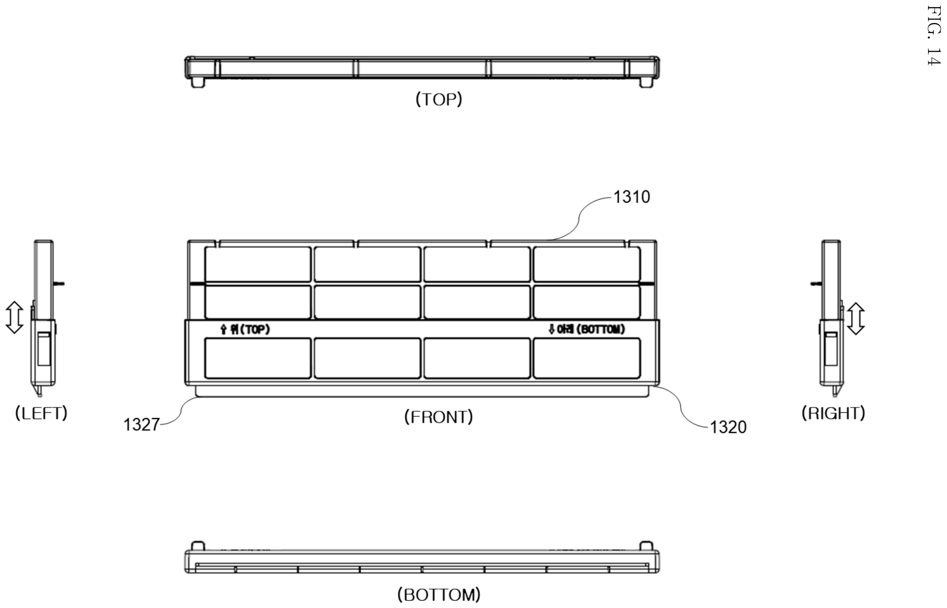

FIG. 13 is a perspective view of a filter member housing 1300 according to at least one embodiment of the present invention. FIG. 14 is a front view, top view, bottom view, and left and right side views of the filter member housing 1300 according to at least one embodiment of the present invention.

In the case of the filter member housing 100 shown in FIG. 1, since the lengthwise cross-section at the lower side of the filter member housing 100 is formed in an angled or rounded shape, when mounted in a general air-circulating electric roaster as shown in FIG. 9, the filter member housing 100 may slide inward into the roaster body, resulting in unstable mounting.

To prevent this, the filter member housing 1300 according to at least one embodiment of the present invention includes an anti-slip member 1327 extending from the lower side of a second member 1120[1320], as shown in FIG. 13.

The anti-slip member 1327 may extend along the entire length of the second member 1120[1320], as shown in FIGS. 13 and 14, or one or more anti-slip members may be provided at selected portions along the length of the second member 1120[1320].

Since other members and functional parts are similar or identical to those of the filter member housing 100 shown in FIG. 4 and the filter member housing 1000 shown in FIG. 10, a detailed description thereof will be omitted.

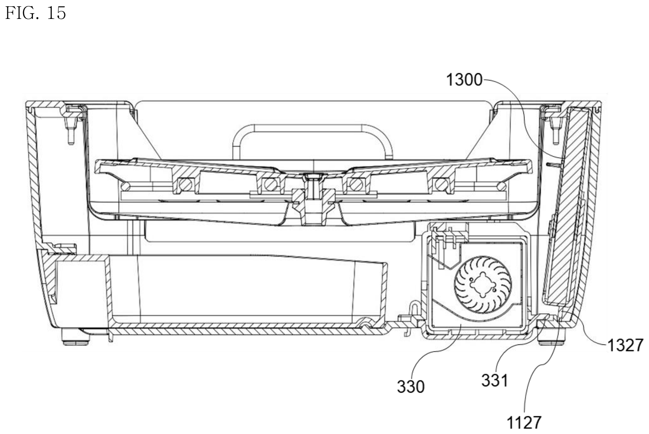

FIG. 15 is a side cross-sectional view of an air-circulating electric roaster with the filter member housing 1300 according to at least one embodiment of the present invention mounted therein.

In the example shown in FIG. 14, the anti-slip member 1327 is formed as a thin plate shape extending from the lower side of the second member 1120[1320], which is inserted between a step 331 of the frame for securing the fan 330 and the lower inner portion of the electric roaster, thereby stably mounting the filter member housing 1300.

Although FIGS. 13 and 14 illustrate the anti-slip member 1327 as a member extending from the lower side of the second member 1120 in a thin plate shape, the anti-slip member 1327 according to at least one embodiment of the present invention may be configured in various shapes to prevent the lower portion of the filter member housing from sliding inward.

As described above, according to at least one embodiment of the present invention, a filter member housing that is easy to install and remove in a confined space and is self-fixed without a separate fixing member can be provided.

Furthermore, with another technical goal to be achieved by the present invention, an air-circulating electric roaster equipped with a filter member housing that is self-fixed without a fixing part to effectively attenuate oil and odors contained in the circulating air of the air-circulating electric roaster can be provided.

Although exemplary embodiments of the present invention have been described for illustrative purposes, those skilled in the art will appreciate that various modifications, additions and substitutions are possible, without departing from the idea and scope of the claimed invention. Accordingly, one of ordinary skill would understand the scope of the claimed invention should not be limited to the above-described embodiments, but should be defined by the claims and equivalents thereof.

### [INDUSTRIAL APPLICABILITY]

The present invention provides a filter member housing that is self-fixed without a separate fixing part and an air-circulating electric roaster including the same, capable of effectively attenuating oil and odors in the circulating air, and thus is applicable to household appliances such as electric grills.

## Claims

1. A filter member housing comprising:
a first member having a first opening formed at a first side along a first direction and a first space for accommodating at least a first side of a filter member through the first opening; and
a second member having a second opening formed at the first side along the first direction so that the first side of the first member is inserted therein, and a second space for accommodating at least a second side of the filter member and the first side of the first member through the second opening, wherein
the first member and the second member are configured to be coupled in a detachable manner, and form a receiving space surrounded by the first space and the second space by inserting a portion of the first side of the first member into the first side of the second member,
the filter member is accommodated in the receiving space by accommodating the first side of the filter member in the first space of the first member or the second side of the filter member in the second space of the second member, and then accommodating the second side of the filter member in the second space of the second member while inserting a portion of the first side of the first member into the first side of the second member or accommodating the first side of the filter member in the first space of the first member while inserting a portion of the first side of the first member into the first side of the second member,
the first member includes a protrusion formed on a portion of the first side that is inserted into the first side of the second member,
the second member includes a groove formed at a position corresponding to the protrusion and configured to allow the protrusion to slide in a second direction orthogonal to the first direction when the portion of the first side of the first member is inserted into the first side of the second member, and
when the filter member is accommodated in the receiving space and the portion of the first side of the first member is inserted into the first side of the second member so that the protrusion is engaged with the groove, the protrusion is positioned at a first end of the groove by an elastic restoring force of the filter member, and when pressure is applied in the second direction to the first member relative to the second member or to the second member relative to the first member, the protrusion moves along the groove toward a second end of the groove, and when the pressure is released, the protrusion returns to the first end of the groove by elastic recovery of the filter member, thereby being fixed in the groove.

2. A filter member housing comprising:
a first member having a first opening formed at a first side along a first direction and a first space for accommodating at least a first side of a filter member through the first opening; and
a second member having a second opening formed at the first side along the first direction so that the first side of the first member is inserted therein, and a second space for accommodating at least a second side of the filter member and the first side of the first member through the second opening, wherein
the first member and the second member are coupled such that a portion of the first side of the first member is inserted into the first side of the second member, thereby defining a receiving space surrounded by the first space and the second space,
the first member and the second member, when coupled, define a lateral opening having one side open along a second direction perpendicular to the first direction,
the filter member is inserted through the lateral opening and accommodated in the receiving space when the first member and the second member are coupled,
the first member includes a protrusion formed on a portion of the first side that is inserted into the first side of the second member,
the second member includes a groove formed at a position corresponding to the protrusion, the groove being configured to allow the protrusion to slide in the second direction when the portion of the first side of the first member is inserted into the first side of the second member, and
when the filter member is accommodated in the receiving space with the first and second members coupled, the protrusion is positioned at a first end of the groove by the elastic force of the filter member, and when pressure is applied to the first member relative to the second member or to the second member relative to the first member along the second direction, the protrusion moves along the groove toward a second end of the groove, and when the pressure is released, the protrusion returns to the first end of the groove by the elastic recovery of the filter member, thereby being fixed in the groove.

3. A filter member housing comprising:
a first member having a first opening formed at a first side along a first direction and a first space for accommodating at least a first side of a filter member through the first opening;
a second member having a second opening formed at the first side along the first direction so that the first side of the first member is inserted therein, and a second space for accommodating at least a second side of the filter member and the first side of the first member through the second opening;
a sliding groove formed in the second member along a second direction perpendicular to the first direction at the first side, the sliding groove allowing sliding and mutual rotation of the first member and the second member when coupled; and
a rotating shaft protrusion formed on the first member and inserted from an inner side of the second member into the sliding groove so as to slide along the sliding groove, wherein
the first member and the second member are mutually rotated about the rotating shaft protrusion to insert a portion of the first side of the first member into the first side of the second member, thereby defining a receiving space surrounded by the first space and the second space,
the filter member is accommodated in the receiving space by inserting its first side into the first space of the first member or its second side into the second space of the second member, and subsequently inserting a portion of the first side of the first member into the first side of the second member,
the first member includes a protrusion formed on a portion of the first side that is inserted into the first side of the second member,
the second member includes a groove formed at a position corresponding to the protrusion, the groove being configured to allow the protrusion to slide along the second direction when the portion of the first side of the first member is inserted into the first side of the second member, and
when the filter member is accommodated in the receiving space and the portion of the first side of the first member is inserted into the first side of the second member so that the protrusion is engaged with the groove, the protrusion is positioned at a first end of the groove by the elastic force of the filter member, and when pressure is applied to the first member relative to the second member or to the second member relative to the first member along the second direction, the protrusion moves along the groove toward a second end of the groove, and when the pressure is released, the protrusion returns to the first end of the groove by the elastic recovery of the filter member, thereby being fixed in the groove.

4. The filter member housing according to any one of claims 1 to 3, wherein the first member or the second member further includes at least one handle for applying pressure to the second member or the first member along the second direction.

5. The filter member housing according to any one of claims 1 to 3, wherein at least one of the first member and the second member includes at least one window opened so that a first surface of the filter member is exposed when the filter member is accommodated in the receiving space.

6. The filter member housing according to any one of claims 1 to 3, wherein
the filter member is accommodated within an installation space having a length in the second direction between a first position, at which the protrusion is positioned at the first end of the groove by the elastic force of the filter member when the filter member is accommodated in the receiving space with the first and second members coupled, and a second position, at which the protrusion moves to the second end of the groove when pressure is applied, and
during installation, the first member or the second member is first inserted into the installation space, followed by pressing the second member against the first member or the first member against the second member, and upon releasing the pressed member, the protrusion returns to the first end of the groove by elastic recovery of the filter member, thereby self-fixing the filter member in the installation space.

7. The filter member housing according to any one of claims 1 to 3, further comprising an anti-slip member formed on a lower side of the second member.

8. An air-circulating electric roaster comprising:
a body;
a heater for generating heat;
a fan disposed inside the body in the first direction;
a cooking plate disposed above the heater and heated by the heat from the heater;
an air intake part having at least one hole for drawing air above the cooking plate into the body;
an air discharge part having at least one hole for discharging air passing through the body toward the top of the cooking plate; and
the filter member housing according to any one of claims 1 to 3.
